Europäisches Patentamt

(19) European Patent Office

Office européen des brevets

(11) Veröffentlichungsnummer: **0 096 754**
**B1**

(12) **EUROPÄISCHE PATENTSCHRIFT**

(45) Veröffentlichungstag der Patentschrift:
27.12.85

(21) Anmeldenummer: 83104837.6

(22) Anmeldetag: 17.05.83

(51) Int. Cl.⁴: **G 01 N 33/18**

(54) Verfahren und Anlage zur kontinuierlichen Aufbereitung von Abwasser sowie seine analytische Untersuchung zur Steuerung von Abwasserreinigungsanlagen.

(30) Priorität: 11.06.82 DE 3222115

(43) Veröffentlichungstag der Anmeldung:
28.12.83 Patentblatt 83/52

(45) Bekanntmachung des Hinweises auf die Patenterteilung:
27.12.85 Patentblatt 85/52

(84) Benannte Vertragsstaaten:
CH DE LI NL SE

(56) Entgegenhaltungen:
EP - A - 0 048 949
DE - A - 2 220 047
DE - A - 2 345 819
GB - A - 1 494 906

(73) Patentinhaber: HOECHST AKTIENGESELLSCHAFT,
Postfach 80 03 20, D-6230 Frankfurt am Main 80 (DE)

(72) Erfinder: Gleisberg, Dietrich, Dr., Falkenweg 12,
D-5042 Erftstadt (DE)
Erfinder: Peantek, Günter, Am Hostert 12,
D-5353 Kommern (DE)
Erfinder: Giehler, Hans-Werner, Am Hofacker 16,
D-5030 Hürth-Gleuel (DE)

**Beschreibung**

Die vorliegende Erfindung betrifft ein Verfahren zur kontinuierlichen Probenahme, Probevorbereitung und Probebehandlung sowie zur analytischen Untersuchung von Abwasser zur Steuerung von Abwasserreinigungsanlagen und eine Anlage zu seiner Durchführung.

Es ist z. B. aus der EP-A-0 048 949 bekannt, Abwasser für analysentechnische Zwecke dadurch aufzubereiten, daß man das Abwasser zunächst über mehrere Sedimentationsstufen laufen läßt, wobei jeweils das an festen Wasserinhaltsstoffen verarmte Abwasser der nächsten Sedimentationsstufe zugeführt wird. Nach der letzten Sedimentationsstufe führt man das vorgeklärte Abwasser einer Bandfiltereinrichtung zu, von der ein für Analysenzwecke aufbereitetes Abwasser abgenommen werden kann. Nach Abnahme des aufbereiteten Wassers wird die Anlage entleert und mit einer neuen Aufbereitung begonnen.

Nachteilig ist dabei, daß die bekannte Anlage wegen ihres Chargenbetriebes einen gleichmäßigen aktuellen Strom des für Analysenzwecke aufbereiteten Abwassers nicht bereitstellen kann. Darüber hinaus benötigen Filtereinrichtungen einen den unterschiedlichen Feststoffbelastungen angepaßten Filterwechsel und damit eine ständige Überwachung.

Es ist daher Aufgabe der vorliegenden Erfindung, ein Verfahren zur kontinuierlichen Aufbereitung von Abwasser, sowie seine analytische Untersuchung zur Steuerung von Abwasserreinigungsanlagen und eine Anlage zu seiner Durchführung anzugeben, bei welchen einem Analysengerät kontinuierlich eine Mischung von für Analysenzwecke aufbereitetem Abwasser und Reagenzlösung zugeführt wird und wobei auf eine Filtrationsstufe bei der Aufbereitung des Abwassers verzichtet wird.

Das wird erfindungsgemäß dadurch erreicht, daß aus der Abwasserreinigungsanlage kontinuierlich Abwasser in eine Hydrozyklonzone gepumpt wird; daß der Ablauf aus der Hydrozyklonzone in die Abwasserreinigungsanlage zurückgeführt wird, während der Überlauf der Hydrozyklonzone in eine Trennzone geführt wird; daß in der Trennzone aufschwimmende und absitzende Feststoffe in die Abwasserreinigungsanlage zurückgeführt werden; daß der vorgeklärte Überstand aus der Trennzone in eine erste Abscheidezone geführt wird; daß der Überlauf der ersten Abscheidezone in einer Mischzone mit einer Reagenzlösung vermischt wird; daß die Mischung in eine Heizzone überführt und bei zur Reaktion, insbesondere Oxidation und/oder Hydrolyse, erforderlicher Temperatur behandelt wird; daß das die Heizzone verlassende Reaktionsprodukt nach Passieren einer Druckausgleichs- und Entgasungszone in eine zweite Abscheidezone strömt; daß der Überlauf der zweiten Abscheidezone in einer Kühlzone auf die zur analytischen Messung erforderliche Temperatur abgekühlt wird; daß der abgekühlte Überlauf der zweiten Abscheidezone ein Analysengerät durchströmt; und daß die vom Analysengerät ermittelten Meßwerte in eine Steuerungseinheit eingegeben werden.

Das Verfahren gemäß der Erfindung kann weiterhin wahlweise auch noch dadurch weitergebildet sein, daß

a) die Mischung in der Heizzone bei Temperaturen von 70 bis 120° C, vorzugsweise von 100 bis 110° C, behandelt wird;

b) der Überlauf der zweiten Abscheidezone auf Temperaturen von 10 bis 40° C, vorzugsweise von 20 bis 30° C, abgekühlt wird.

Eine Anlage zur Durchführung des erfindungsgemäßen Verfahrens besteht aus einem Trichter, einem Hydrozyklon, einem Trenngefäß, einem ersten geschlossenen Abscheider, einer Mischeinrichtung, mindestens einem Reagenzien-Behälter, einem Heizbehälter, einem zweiten geschlossenen Abscheider, einem Kühlbehälter und einem Analysengerät, wobei die Beaufschlagung des Hydrozyklons mit Abwasser durch den Trichter, sowie über eine Rohrleitung und eine Pumpe erfolgt, und wobei der Überlauf aus dem Hydrozyklon über eine Leitung in das Trenngefäß gelangt, und wobei eine Pumpe vorgeklärtes Abwasser aus dem Trenngefäß über den ersten geschlossenen Abscheider ansaugt und in die Mischeinrichtung drückt, und wobei gleichzeitig eine Pumpe Reagenzlösung aus dem Reagenzien-Behälter ansaugt und in die Mischeinrichtung drückt, und wobei die Mischung durch Durchströmen einer im Heizbehälter befindlichen Wärmeaustauscher- und Verweilspirale erhitzt und über ein Standrohr entgast wird, und wobei die entgaste heiße Mischung über eine Leitung in den zweiten geschlossenen Abscheider geführt wird, und wobei der Überlauf aus dem zweiten geschlossenen Abscheider durch eine im Kühlbehälter befindliche Spirale geführt wird, und wobei die abgekühlte Mischung über eine mit Querschnittserweiterung versehene Leitung in das Analysengerät einläuft, und wobei die Mischung nach erfolgter Analyse durch einen Ablauf aus dem Analysengerät austritt.

Die Anlage zur Durchführung des erfindungsgemäßen Verfahrens kann wahlweise auch noch dadurch ausgestaltet sein, daß

c) der Trichter mit seiner Mündung gegen den Abwasserstrom liegend in einem Abwasserkanal angeordnet ist;

d) das Trenngefäß in seinem oberen Bereich eine Überlaufrinne aufweist;

e) am auslaufseitigen Ende des Trenngefäßes ein Heber angeordnet ist;

f) die geschlossenen Abscheider mit Ventilen versehene Ablaufleitungen aufweisen;

g) das Analysengerät ein Photometer ist;

h) im auslaufseitigen Ende des Hebers ein Ventil angeordnet ist.

Mit dem Verfahren gemäß der Erfindung werden aus dem Abwasserstrom Feststoffe unterschiedlicher Größe und Beschaffenheit, welche insbesondere analytische Bestimmungen auf der Grundlage der Photometrie stören, entfernt, bevor das inzwischen mit der Reagenzlösung versetzte Abwasser in das Analysengerät eintritt.

Beim erfindungsgemäßen Verfahren wird der Probenstrom kontinuierlich, beispielsweise photometrisch in einer Küvette, gemessen. Die Belastung des Abwassers mit gelösten, kolloiden und feindispersen Stoffen läßt sich damit automatisch bestimmen und das Meßsignal zur Steuerung einsetzen. Beispielsweise läßt sich der Phosphatgehalt des Abwassers bestimmen und das Analysenergebnis zur Steuerung der Fällmitteldosierung einsetzen oder die Bestimmung des CSB-Wertes (Chemischer Sauerstoffbedarf) läßt sich zur Steuerung der Luftmenge sowie der Abwasser- und Schlammströme in Belebungsbekken verwenden.

Bei der Anlage zur Durchführung des erfindungsgemäßen Verfahrens kann die Mischeinrichtung eine Spirale, eine Mischdüse oder eine Wirbeleinrichtung sein.

Der Querschnitt der Leitungen, welche bei der Anlage zur Durchführung des erfindungsgemäßen Verfahrens die einzelnen Anlagenteile miteinander verbinden, kann zur Verhinderung von Verstopfungen in Fließrichtung geringfügig erweitert sein.

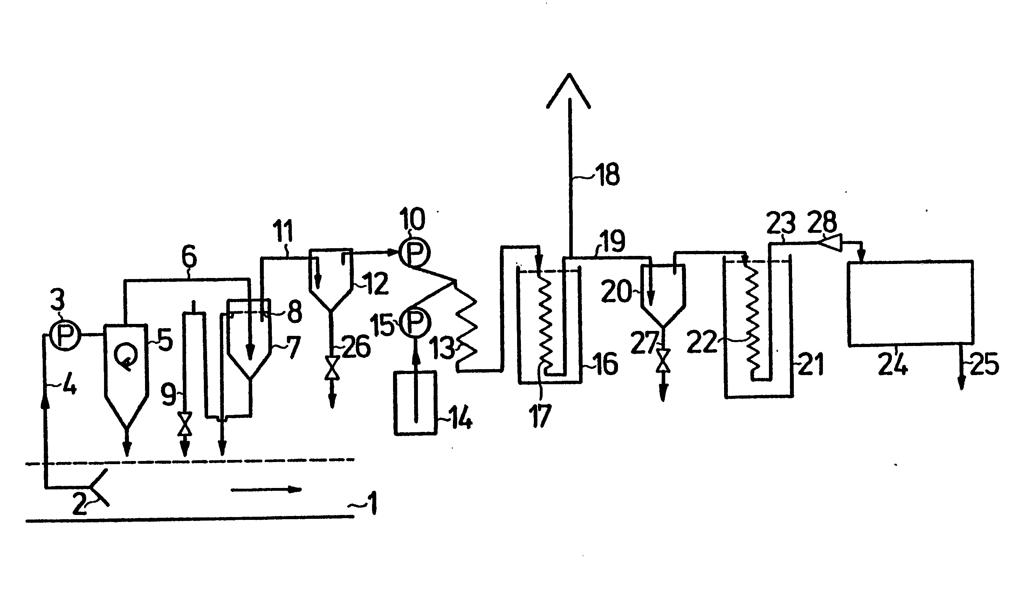

In der beigefügten Zeichnung ist eine Anlage zur Durchführung des erfindungsgemäßen Verfahrens schematisch dargestellt.

Durch einen im Abwasserkanal 1 gegen den Abwasserstrom liegenden Trichter 2 wird das Abwasser durch die Pumpe 3 über die Rohrleitung 4 in den Hydrozyklon 5 gepumpt. Der Überlauf aus dem Hydrozyklon 5 gelangt über die Leitung 6 in das Trenngefäß 7, aus welchem die aufschwimmenden und absinkenden Feststoffe über die Überlaufrinne 8 bzw. den Heber 9 ablaufen. Die Pumpe 10 saugt aus dem oberen Drittel des Trenngefäßes 7 über die Leitung 11 und den ersten geschlossenen Abscheider 12 vorgeklärtes Abwasser an und drückt es in die Mischeinrichtung 13, in welche gleichzeitig Reagenzlösung aus dem Reagenzien-Behälter 14 durch die Pumpe 15 hineingepumpt wird. Die in der Mischeinrichtung 13 erhaltene Mischung durchströmt eine in einem Heizbehälter 16 angeordnete Wärmeaustauscher- und Verweilspirale 17. Der Druckausgleich und die Entgasung der heißen Mischung erfolgt über das Standrohr 18. Die heiße Mischung läuft über die Leitung 19 in den zweiten geschlossenen Abscheider 20 ein. Der Überlauf aus dem Abscheider 20 durchströmt eine in einem Kühlbehälter 21 angeordnete Spirale 22, bevor er abgekühlt über die mit Querschnittserweiterung 28 versehene Leitung 23 in das Analysengerät 24 einläuft. Nach erfolgter Analyse wird die Flüssigkeit über den Ablauf 25 aus dem Analysengerät 24 ausgeschleust.

Die Menge des Bodenablaufes von Trenngefäß 7 sowie von den beiden geschlossenen Abscheidern (12, 20) läßt sich durch im Heber 9 bzw. in den Ablaufleitungen (26, 27) angeordnete Ventile einstellen.

## Patentansprüche

1. Verfahren zur kontinuierlichen Probenahme, Probevorbereitung und Probebehandlung sowie zur analytischen Untersuchung von Abwasser zur Steuerung von Abwasserreinigungsanlagen, in dem aus der Abwasserreinigungsanlage kontinuierlich Abwasser in eine Hydrozyklonzone gepumpt wird; der Ablauf aus der Hydrozyklonzone in die Abwasserreinigungsanlage zurückgeführt wird, während der Überlauf der Hydrozyklonzone in eine Trennzone geführt wird; in der Trennzone aufschwimmende und absitzende Feststoffe in die Abwasserreinigungsanlage zurückgeführt werden; der vorgeklärte Überstand aus der Trennzone in eine erste Abscheidezone geführt wird; der Überlauf der ersten Abscheidezone in einer Mischzone mit einer Reagenzlösung vermischt wird; die Mischung in eine Heizzone überführt und bei zur Reaktion, insbesondere Oxidation und/oder Hydrolyse, erforderlicher Temperatur behandelt wird; das die Heizzone verlassende Reaktionsprodukt nach Passieren einer Druckausgleichs- und Entgasungszone in eine zweite Abscheidezone strömt; der Überlauf der zweiten Abscheidezone in einer Kühlzone auf die zur analytischen Messung erforderliche Temperatur abgekühlt wird; der abgekühlte Überlauf der zweiten Abscheidezone ein Analysengerät durchströmt; und die vom Analysengerät ermittelten Meßwerte in eine Steuerungseinheit eingegeben werden.

2. Verfahren nach Anspruch 1, dadurch gekennzeichnet, daß die Mischung in der Heizzone bei Temperaturen von 70 bis 120°C, vorzugsweise von 100 bis 110°C, behandelt wird.

3. Verfahren nach Anspruch 1 oder 2, dadurch gekennzeichnet, daß der Überlauf der zweiten Abscheidezone auf Temperaturen von 10 bis 40°C, vorzugsweise von 20 bis 30°C, abgekühlt wird.

4. Anlage zur Durchführung des Verfahrens nach den Ansprüchen 1 bis 3, bestehend aus einem Trichter (2), einem Hydrozyklon (5), einem Trenngefäß (7), einem ersten geschlossenen Abscheider (12), einer Mischeinrichtung (13), mindestens einem Reagenzien-Behälter (14), einem Heizbehälter (16), einem zweiten geschlossenen Abscheider (20), einem Kühlbehälter (21) und einem Analysengerät (24), wobei die Beaufschlagung des Hydrozyklons mit Abwasser durch den Trichter, sowie über eine Rohrleitung (4) und eine Pumpe (3) erfolgt, und wobei der Überlauf aus dem Hydrozyklon (5) über eine Leitung (6) in das Trenngefäß (7) gelangt, und wobei eine Pumpe (10) vorgeklärtes Abwasser aus dem Trenngefäß (7) über den ersten geschlossenen Abscheider (12) ansaugt und in die Mischeinrichtung (13) drückt, und wobei gleichzeitig eine Pumpe (15) Reagenzlösung aus dem Reagenzien-Behälter (14) ansaugt und in die Mischeinrichtung (13) drückt, und wobei die Mischung durch Durchströmen einer im Heizbehälter (16) befindlichen Wärmeaustauscher- und Verweilspirale (17) erhitzt und über ein Standrohr (18) entgast wird, und wobei die entgaste heiße Mischung über eine Leitung (19) in den zweiten geschlossenen Abscheider (20) geführt wird, und wobei der Überlauf aus dem zweiten geschlossenen Abscheider (20) durch eine im Kühlbehälter (21) befindliche Spirale (22) geführt wird, und wobei die abgekühlte Mischung über eine mit Querschnittserweiterung (28) versehene Leitung (23) in das Analysengerät (24) einläuft, und wobei die Mischung nach erfolgter Analyse durch einen Ablauf (25) aus dem Analysengerät (24) austritt.

5. Anlage nach Anspruch 4, dadurch gekennzeichnet, daß der Trichter (2) mit seiner Mündung gegen den Abwasserstrom liegend in einem Abwasserkanal (1) angeordnet ist.

6. Anlage nach Anspruch 4 oder 5, dadurch gekennzeichnet, daß das Trenngefäß (7) in seinem oberen Bereich eine Überlaufrinne (8) aufweist.

7. Anlage nach einem der Ansprüche 4 bis 6, dadurch gekennzeichnet, daß am auslaufseitigen Ende des Trenngefäßes (7) ein Heber (9) angeordnet ist.

8. Anlage nach einem der Ansprüche 4 bis 7, dadurch gekennzeichnet, daß die geschlossenen Abscheider (12, 20) mit Ventilen versehene Ablaufleitungen (25, 27) aufweisen.

9. Anlage nach einem der Ansprüche 4 bis 8, dadurch gekennzeichnet, daß das Analysengerät (24) ein Photometer ist.

10. Anlage nach Anspruch 7, dadurch gekennzeichnet, daß im auslaufseitigen Ende des Hebers (9) ein Ventil angeordnet ist.

## Claims

1. Process for continuously sampling, preparing and treating as well as analytically investigating waste water and controlling a waste water purification plant by: continuously pumping waste water coming from the waste water purification plant into a hydrocyclone zone; recycling effluent matter coming from the hydrocyclone zone into the waste water purification plant, matter coming from the overflow of the hydrocyclone zone being introduced into a separating zone; recycling solid matter floating or depositing in the separating zone into the waste water purification plant; introducing pre-purified supernatant matter from the separating zone into a first precipitating zone; mixing the overflow matter from the first precipitating zone with a test solution in a mixing zone; introducing the mixture into a heating zone and treating it at the temperature necessary for effecting the reaction, especially oxidation and/or hydrolysis; passing the reaction product coming from the heating zone through a pressureequalizing and degassifying zone into a second precipitating zone; cooling the overflow matter coming from the second precipitating zone in a cooling zone to the temperature necessary for analyzing; passing the cooled overflow matter from the second precipitating zone through an analyzer; and feeding the test values determined by the analyzer to a control unit.

2. Process as claimed in claim 1, wherein the mixture is treated in the heating zone at temperatures of 70 to 120°C, preferably 100 to 110°C.

3. Process as claimed in claim 1 or 2, wherein the overflow matter coming from the second precipitating zone is cooled to temperatures of 10 to 40°C, preferably 20 to 30°C.

3. Apparatus for carrying out the process as claimed in claims 1 to 3 comprised of a funnel (2), a hydrocyclone (5), a separator (7), a first closed precipitator (12), a mixer (13), at least one reagent container (14), a heater (16), a second closed precipitator (20) a cooler (21) and an analyzer (24), the hydrocyclone (5) receiving the waste water through the funnel (2), a conduit (4) and a pump (3); overflow matter coming from the hydrocyclone (5) being introduced through a conduit (6) into the separator (7); pre-purified waste water coming from the separator (7) being passed by means of a pump (10) through the first closed precipitator (12) into the mixer (13); a test solution coming from the reagent container (14) being simultaneously passed into the mixer (13) by means of a pump (15); the mixture flowing through a heat exchanger and sojourn spiral (17) disposed in the heater (16) becoming heated and degassed in a standpipe (18); the degassed hot mixture being introduced through a conduit (19) into the second closed precipitator (20); overflow matter coming from the second closed precipitator (20) being passed through a spiral (22) in cooler (21); cooled mixture travelling through a conduit (23) with a cross-sectionally widened portion (28) into the analyzer (24); and analyzed mixture issuing from the analyzer (24) through an outlet (25).

5. Apparatus as claimed in claim 4, wherein the funnel (2) is disposed in a waste water channel (1) with the funnel mouth opening oppositely with respect to the direction of flow of the waste water.

6. Apparatus as claimed in claim 4 or 5, wherein the separator (7) is formed in its upper region with an overflow channel (8).

7. Apparatus as claimed in any of claims 4 to 6, wherein the effluent outlet of separator (7) is provided with a siphon (9).

8. Apparatus as claimed in any of claims 4 to 7, wherein the closed precipitators (12, 20) are formed with valved effluent outlets (26, 27).

9. Apparatus as claimed in any of claims 4 to 8, wherein the analyzer (24) is a photometer.

10. Apparatus as claimed in claim 7, wherein the siphon (9) has a valve secured to its effluent outlet.

## Revendications

1. Procédé de prélèvement, de préparation et de traitement en continu ainsi que d'examen analytique des eaux résiduaires et de contrôle d'installations de purification des eaux résiduaires en dirigeant en continu à l'aide d'une pompe les eaux résiduaires depuis l'installation de purification dans une zone d'hydrocyclone; en recyclant l'effluent de la zone d'hydrocyclone dans l'installation de purification des eaux résiduaires, tandis que la matière sortant par le trop-plein de la zone d'hydrocyclone est introduite dans une zone de séparation; en recyclant la matière flottante ou déposée dans la zone de séparation dans l'installation de purification des eaux résiduaires; en introduisant la matière surnageante prépurifiée depuis la zone de séparation dans une première zone de précipitation; en mélangeant la matière sortant par le trop-plein de la première zone de précipitation avec une solution d'essai dans une zone de mélange; en introduisant le mélange dans une zone de chauffage et en le traitant à la température nécessaire pour la réaction, en particulier l'oxydation et/ou l'hydrolyse; en passant le produit de réaction sortant de la zone de chauffage par une zone d'égalisation de pression et de dégazage dans une seconde zone de précipitation; en refroidissant la matière sortant par le trop-plein de la seconde zone de précipitation dans une zone de refroidissement à la température nécessaire à pour l'analyse; en passant la matière de trop-plein refroidie de la seconde zone de précipitation par un dispositif d'analyse; et en communiquant les valeurs mesurées par le dispositif d'analyse à une unité de contrôle.

2. Procédé selon la revendication 1, caractérisé en ce que l'on traite le mélange dans la zone de chauffage à des températures de 70 à 120°C, de préférence à 100 à 110°C.

3. Procédé selon la revendication 1 ou 2, caractérisé en ce que l'on refroidit la matière de trop-plein de la second zone de précipitation à des températures de 10 à 40°C, de préférence 20 à 30°C.

4. Appareil pour la mise en œuvre du procédé selon les revendications 1 à 3 constitué par un entonnoir (2), un hydrocyclone (5), un séparateur (7), un premier dispositif de précipitation fermé (12), un mélangeur (13), au moins un récipient à réactif (14), un récipient de chauffage (16), un second dispositif de précipitation fermé (20), un réfrigérant (21) et un dispositif d'analyse (23), les eaux résiduaires étant amenées à l'hydrocyclone (5) par l'entonnoir (2), un conduit (4) et une pompe (3); la matière de trop-plein de l'hydrocyclone (5) étant introduite à travers un conduit (6) dans le séparateur (7); les eaux résiduaires prépurifiées du séparateur (7) étant passées à l'aide d'une pompe (10) par le premier dispositif de précipitation fermé dans le mélangeur (13); une solution d'essai provenant du récipient à réactif (14) étant simultanément passée dans le mélangeur (13) par l'intermédiaire d'une pompe (15); le mélange passant par une spirale de séjour et d'échange de chaleur (17) disposés dans le récipient de chauffage étant chauffé et dégazifié dans un tube plongeant (18); le mélange chaud dégazifié étant introduit par un conduit (19) dans le second dispositif de précipitation fermé (20); la matière de trop-plein du second dispositif de précipitation fermé (20) étant passée par une spirale (22) agencée dans le réfrigérant (21); le mélange refroidi s'écoulant par un conduit (23) muni d'une partie évasée en diamètre (28) dans le dispositif d'analyse (24); et le mélange analysé sortant du dispositif d'analyse (24) par une sortie (25).

5. Appareil selon la revendication 4, caractérisé en ce que l'entonnoir (2) est disposé dans un conduit d'évacuation des eaux résiduaires (1), la bouche de l'entonnoir débouchant en direction opposité à la direction d'écoulement des eaux résiduaires.

6. Appareil selon la revendication 4 ou 5, caractérisé en ce que le séparateur (7) est muni dans sa partie supérieure d'un conduit de trop-plein (8).

7. Appareil selon l'une des revendication 4 à 6, caractérisé en ce qu'un siphon (9) is pourvu au côté d'effluent du séparateur (7).

8. Appareil selon l'une des revendications 4 à 7, caractérisé en ce que les dispositifs de précipitation fermés (12, 20) sont pourvus de conduits de sortie (26, 27) munis de vannes.

9. Appareil selon l'une des revendications 4 à 8, caractérisé en ce que le dispositif d'analyse (24) est un photomètre.

10. Appareil selon la revendication 7, caractérisé en ce qu'une vanne est agencée au côté d'effluent du siphon 9.

0 096 754